# EUROPEAN PATENT APPLICATION

(11) **EP 2 087 900 A1**
(43) Date of publication of application: **12.08.2009**
(21) Application number: 08002222.1
(22) Date of filing: 07.02.2008
(51) Int. Cl.: A61K 36/16, A61P 25/28

(54) **Ginkgo biloba extract with a standardised ginkgo flavone glycosides content deprived of the paf-antogonist terpenic fraction, and compostions containing it, for the prevention and treatment of circulatory, cognitive, geriatric and sensory disorders**

(71) Applicant: Velleja Research SRL, 20122 Milano (IT)
(72) Inventor: Di Pierro, Francesco, 29010 Pontenure (PC) (IT)
(74) Representative: Minoja, Fabrizio

(57) **Abstract**

Disclosed is a *Ginkgo biloba* extract substantially devoid of PAF-antagonist activity.

## Description

The present invention relates to a *Ginkgo biloba* extract devoid of PAF-antagonist activity, a process for its preparation, and compositions containing said extract.

### Background to the invention

Extracts of *Ginkgo biloba,* one of the oldest medicinal plants, are used for a wide variety of nutraceutical, pharmaceutical and cosmetic applications. The active ingredients of the extracts have long been known and studied: in addition to ginkgo flavone glycosides (in particular glycoside derivatives of kaempferol, quercetin, rutin, luteolin, isorhamnetin and myricetin), diterpene compounds (such as ginkgolides A, B, C J and M) and sesquiterpene compounds (bilobalide) are usually also present. Other ingredients, such as ginkgolic acids (also known as anacardic acids), may also be present. *Ginkgo biloba* extracts are mainly used in solid oral forms for the treatment of Alzheimer's disease, *claudicatio intermittens*, to improve the cognitive functions in cerebrovascular insufficiency, in disorders associated with poor peripheral circulation (including impotence) and in sensory disorders connected with the sight and hearing.

The industrial processes used to prepare *Ginkgo biloba* extracts must comply with specific regulatory requirements and guarantee qualitative and quantitative uniformity of composition. The extracts must therefore be "standardised" by means of analytical characterisation which complies, for example, with the specifications of Official Pharmacopoeias, where present, or in any event guarantee the desired biological efficacy and the substantial absence of side effects.

For example, EP 360556 discloses a purified extract of *Ginkgo biloba* obtainable by extraction from the leaves with a mixture of lower alcohols C3-C6 and aromatic hydrocarbons C6-C8, which contains 22 to 26% of ginkgo flavone glycoside, under 10% of proanthocyanidins, 2.5 to 4.5% of ginkgolides, 2.5 to 4.5% of bilobalide, and is substantially devoid of lipophilic substances (ginkgolic acids or alkylphenols).

The preparation of an extract with a very similar composition by extraction with a mixture of water and alcohols is described in EP 1089748.

US 6022889 discloses a *Ginkgo biloba* extract with an enriched terpene fraction, in particular enriched with bilobalide, which is useful as an anxiolytic and antidepressant.

EP 822825 discloses a *Ginkgo biloba* extract characterised by a higher ginkgo flavone glycoside content (28 to 32%) and a terpene content below 0.5%. The terpenes are removed at the stage of extraction with alkyl esters, in particular ethyl acetate. The extract is used as a flavouring agent, and the removal, or rather the reduction of the terpenes is designed to improve the organoleptic characteristics of the extract, no pharmacological significance being attributed to the reduction of the terpenic fraction.

EP 934074 describes the use of an extract similar to the one described in EP 822825, with a terpenic content of under 1% and preferably under 0.5%, in combination with ceramides, for the treatment of dental and stomatological disorders.

The extracts most widely available on the market are listed in the scientific documentation with the code Egb 761 and the trademark Ginkgoselect® (Indena).

The doses of standardised dried extract most commonly used in treatment range between 40 and 360 mg a day. Most commonly, the doses range between 120 and 240 mg/day, the higher dose of 360 mg/day being reserved for initial treatments and patients suffering from serious dementia. The dose is usually divided into 3 treatments a day.

A common characteristic of all the known extracts is the starting material, consisting of dried leaves which, in addition to the above-mentioned ingredients, contain a dimeric flavone fraction consisting of amentoflavone, bilobetin, sequoiaflavone, ginkgetin, isoginkgetin and sciadopitysin, which is not normally found in the end product.

The majority of the extracts currently available contain the terpenic fraction in substantial quantities (up to 6% of the extract), to the extent that it is one of the analytical parameters used for the standardisation and Pharmacopoeia codification. Although this fraction is believed to contribute to the known activities of Ginkgo extracts, it also possesses a PAF (Platelet-Activating Factor)-antagonist action.

As PAF is an important mediator of coagulation processes, competitive antagonism towards its receptor generates anti-blood clotting effects which are experimentally measurable by analysing bleeding times. These times are increased to a dose-dependent extent as a result of administration of the product. For example, in laboratory animals, bleeding times are increased by 5 to 20% following oral administration of 50-300 mg/kg of a conventional Ginkgo extract.

If the extract is administered in combination with anti-blood clotting drugs (NSAIDs, warfarin, etc.) the times are increased much more, by up to 150%, to a dose-dependent extent. The problem is also encountered in clinical practice. Various cases of haemorrhage caused by interaction with drugs that inhibit platelet aggregation are described in the literature. For example, cases of spontaneous subarachnoid haemorrhage have been reported in patients aged around 70 years old who took *Ginkgo biloba* extracts in combination with acetylsalicylic acid: the bleeding proved to be reversible when the treatment was discontinued. A single case of cerebral haemorrhage has been reported in a patient aged 71 who underwent concomitant administration of *Ginkgo biloba* and ibuprofen. Other clinical cases demonstrate that the administration of *Ginkgo biloba* extract has been associated with cases of non-lethal subarachnoid haemorrhage in patients undergoing chronic treatment with warfarin. In these latter cases, the interaction seems to have both a pharmacodynamic and a pharmacokinetic basis. *Ginkgo biloba* extract inhibits the microsomal metabolism of warfarin, due to action on isoenzymes CPY2C9 and CYP3A4 of cytochrome P450. Finally, cases of spontaneous haemorrhage of the iris have been reported in patients already under treatment with acetylsalicylic acid or ergotamine, who were treated simultaneously with *Ginkgo biloba extracts*.

The combination of *Ginkgo biloba* extracts and anticoagulant or anti-blood clotting drugs must therefore be avoided. Treatment with *Ginkgo biloba* extract should also be suspended at least 36 hours before any surgery (including tooth extractions).

The PAF-antagonist action of the terpenic fraction is not only dangerous in the event of co-administration with anti-blood clotting, but also constitutes a major pharmacological limitation in geriatric practice, as elderly patients are commonly treated with anti-blood clotting to reduce the cardiovascular risk (thrombosis, heart attack and stroke) or inhibit tumour metastasis. The administration of *Ginkgo biloba* extracts to elderly patients is particularly desirable in order to counteract the neuronal degeneration processes that lead to various symptoms, ranging from loss of short-term memory to more or less evident cognitive decay and decline of the learning processes, and finally to serious, full-blown signs of dementia (senile or multi-infarct) and/or Alzheimer's disease.

The need for a *Ginkgo biloba* extract that maintains the activities typical of the extract but has no PAF-antagonist activity is therefore particularly felt.

### Description of the invention

It has now been found that is possible to obtain a *Ginkgo biloba* extract having a ginkgo flavone glycoside and proanthocyanidin content substantially similar to that of conventional extracts, in particular extracts obtainable with the process described in EP 360556, but substantially devoid of the PAF-antagonist (terpenic) fraction.

"Substantially devoid" means a terpene content of under 0.1%, preferably under 0.01%, and even more preferably under 0.001%.

The validated analysis method used to analyse the terpenic fraction (ginkgolides A, B, C, J, M and bilobalide) is reported in Croom E, Pace R, Paletti A, Sardone N, Gray D. Single-laboratory validation for the determination of terpene lactones in *Ginkgo biloba* dietary supplement crude materials and finished products by high-performance liquid chromatography with evaporative light-scattering detection. J AOAC Int. 2007 May-Jun;90(3):647-58.

The extract according to the invention can be obtained by repeated extractions of a conventional extract in solvents chosen from alkyl esters, ketones, chlorinated hydrocarbons, supercritical CO₂, hexane, and ethanol-water mixtures with an ethanol content of between 70 and 95% v/v. In the latter case, however, the preparation will need to be reconstituted with the ginkgo flavone glucoside fraction partly extracted from the solvent. The preferred extraction solvent is ethyl acetate. Inert, non-toxic material such as maltodextrins, dietary fibres, cellulose, sugars and/or polyols can be added to the extract devoid of the terpenic fraction.

An example of the process for the preparation of the extract according to the invention, hereinafter referred to by the code VR-456, involves extraction from dehydrated *Ginkgo biloba* leaves with a 6:4 mixture of acetone:water; the product obtained, after discarding the fraction not soluble in the solvent, is then washed in countercurrent with n-hexane; the fraction soluble in n-hexane is discarded, and the derivative is concentrated with water and then centrifuged to discard the solid precipitate; the derivative obtained is extracted in countercurrent with a 4:1 mixture of n-butanolaoluene; the fraction soluble in water is discarded, the derivative is concentrated, and the residual solvents eliminated; the terpene fraction is removed from the dehydrated product thus obtained and redissolved in water with 10-15 (preferably 15) washes in ethyl acetate (or other solvents able to extract lipophilic matrices) for a total duration of 15-180 min (preferably 30 min) at a temperature of between 15 and 45°C (preferably 25°C); it is then concentrated again and dried, and the residual solvent is eliminated if still present. Specifically, and merely by way of example, for 1 kg of starting extract, not yet stripped of terpenes by washing in ethyl acetate, 10 volumes of water (10 litres) in which the extract is dissolved, and 75 volumes (75 litres) of ethyl acetate to remove the terpene fraction are required. Using this manufacturing process, and after standardisation with excipients if necessary, the extracted derivative obtained claims the following titration and standardisation: 22-26% of ginkgo flavone glycoside, < 0.1 % of terpenes, < 5 ppm of ginkgolic acids. The terpene content can be further reduced to values below 0.01 % by a further 15 washes in ethyl acetate. A further 15 washes in the solvent will give analytical values below 0.001% for the terpene fraction. Whatever the % setting of the terpene fraction (from < 0.1% to < 0.001 %), the product is then mixed with a suitable proportion of inert material (maltodextrins, polyols, cellulose, etc.), and reconstituted. The same product can also be obtained by extraction with ethanol and ethanol:water with various washes before eliminating the terpene fraction with ethyl acetate or CO₂ or hexane, or butyl acetate, CH2Cl2 and methyl ethyl ketone, the subsequent washes and the ratios between the solvents used, and the times and temperature used for the process, remaining unchanged.

The extract thus reconstituted (VR-456) and devoid of the PAF-antagonist fraction wholly maintains its useful vasculokinetic, antigeriatric, procognitive, prosensory and antidementia properties, which are similar to those of the native extract. Unlike the native extract, VR-456 is devoid of any effect associated with PAF-antagonism: it therefore possesses no anti-platelet aggregation action, anti-asthma action, anti-allergic action, anti-migraine action or anti-headache action, and anti-inflammatory properties most evident at ocular and intestinal level.

In particular, the product does not manifest any additive and/or synergic effect if co-administered with platelet anti-blood clotting (NSAIDs, warfarin, etc.). In co-administration, the bleeding time measured in the rat is always similar to that of the controls treated with the anti-blood clotting only. Consequently, VR-456 administered alone does not cause any significant increase in bleeding times; in the same way, when administered together with a platelet anti-blood clotting, it does not increase the bleeding times due to the anti-blood clotting. The same type of result was obtained by measuring bleeding times in healthy volunteers.

VR-456 still retains the "*cognition-enhancing*" activity described for the native extract, and can therefore be used successfully in the treatment of elderly people suffering from mild, moderate or evident signs of cerebral/cognitive deficiency, connected with a simple reduction in memory capacity (especially short-term memory) and with a more serious situation of multi-infarct dementia or Alzheimer's disease. Typically, elderly patients are given chronic treatment with drugs that reduce the coagulation risk (such as "cardio-aspirin®"). In such case the use of the native extract is dangerous, because the anti-blood clotting effect of the drug used is combined with the PAF-antagonist action of the terpenic fraction present in the extract. However, the use of VR-456 allows safe treatment to be given. Finally, the dose of VR-456 is the same as the normal dose of conventional *Ginkgo biloba* extracts: 40 mg to 360 mg/day, divided into 3 daily administrations; more specifically, the product should be administered at the dose of 360 mg/day at the start of the treatment (3 months) in cases where severe dementia according to the ADAS-Cog and GERRI scale is diagnosed. More specifically, 240 mg/day could be administered in the continuance of the treatment (the next 9 months). After one year's treatment, the dose could be reduced to 120 mg/day. Otherwise, in cases of slight cognitive deficit not necessarily associated with Alzheimer's disease or multi-infarct dementia, but characterised by a simple memory difficulty (even in young patients), the dose of 120 mg/day (40 mg x 3) could be given.

VR-456 could also be enhanced in terms of oral bioavailability by the processes with which it is made:
1) as a liposome where the ratio between phospholipids (carrier unit) and VR-456 ranges between 1:10 and 1:10,000;
2) as a phytosome constituted in aprotic solvent due to the stoichiometric interaction in aprotic solvent of 1 part of the product with 1 or 2 parts of distearoylphosphatidylcholine with various degrees of purity (preferably between 30 and 100%); phospholipids wherein the fatty acid chains differ in terms of stearic acid, and consequently have a carbon skeleton from C3 to C22, could also be used in this case. In the same way, the kinetic characteristics of VR-456 could be implemented by procedures of:
3) co-grinding and ter-clatration based on mixing in offset grinders. The mixtures will preferably consist of VR-456 and cyclodextrins, or VR-456 and dehydrogenated or non-dehydrogenated oils (preferably palm oil).

VR-456 is consequently the ideal product in all situations in which a vasculokinetic, antidementia, ant-geriatric or prosensory action is required, in the presence of an existing, essential anti-blood clotting treatment. The extract could obviously be further advantageously used for persons other than geriatric patients (such as young people engaged in difficult memorising, working or sports tasks), either alone or in combination with other compounds that enhance its vasculokinetic, anti-dementia or prosensory action. For example, in erectile dysfunction, VR-456 (120 mg/day) could be advantageously associated with L-arginine (250 mg/day) and *Tribulus terrestris* dried extract (100 mg/day); in the prevention and treatment of poor concentration and memorisation, the association between VR-456 (120 mg/day), phosphatidylserine (50 mg/day) and *Panax ginseng* dried extract (150 mg/day) will be advantageous, whereas in tinnitus, VR-456 could be associated with ingredients with an antioxidant activity or sedative/tranquillising activity, or ingredients with anti-premenstrual syndrome and anti-menopause activity such as herbal derivatives containing coumarins (*Melilotus officinalis, Aesculus hyppocastanum*), leucocyanidins (*Vitis vinifera*), anthocyanins (*Vaccinium myrtillus*), flavonolignans (*Silybum marianum*), catechins and epicatechins (*Camellia sinensis*); compounds with a hormonal and/or adaptogenic action (extract of *Tribulus terrestris, Panax ginseng, Panax quinquefolium, Eleuterococcus senticosus, Echinacea spp.* or *Astragalus membranaceus*); compounds with cognitive/sensory effects such as L-serine (including derivatives and transporters thereof) and choline (including derivatives and transporters thereof); vitamins, trace elements, enzymes, and metabolic intermediates (such as lactic acid); compounds with sedative, tranquillising, spasmolytic, antipanic or anxiolytic activity (such as extracts of *Valeriana officinalis, Passiflora incarnata, Crategus spp., Matricaria camomile*, tryptophan, *Griffonia simplicifolia, kawa-kawa, Hypericum perforatum*); compounds useful in the treatment of premenstrual syndrome and menopause syndrome (*Vitex agnus castus, Glycine max, Cimicifuga racemosa, Trifolium pratense,* pyridoxine and magnesium).

### EXAMPLES OF FORMULATIONS

| 1) Film-coated tablets: | |
|---|---|
| VR-456 | 120 mg |
| Arginine | 100 mg |
| Glutamic acid | 100 mg |
| Proline | 100 mg |
| Ornithine alpha-ketogluturate | 100 mg |
| Citrulline | 100 mg |
| Microcel | 200 m |
| Dicafos | 200 mg |
| Vegetable magnesium stearate | 40 mg |
| PVP CL | 40 mg |
| Silicon dioxide | 20 mg |
| Shellac | 50 mg |

| 2) "0" Format capsules: | |
|---|---|
| VR-456 | 120 mg |
| *Tribulus terrestris* | 250 mg |
| Arginine | 50 mg |
| Ornithine alpha-ketogluturate | 50 mg |
| Talc | 10 mg |
| Microcel | 50 mg |

| 3) Sachets: | |
|---|---|
| VR-456 | 120 mg |
| Arginine | 500 mg |
| Glutamic acid | 500 mg |
| Proline | 500 mg |
| Ornithine alpha-ketogluturate | 500 mg |
| Citrulline | 500 mg |
| Fructose | 1000 mg |
| Methocel E5 | 20 mg |
| Aerosil | 50 mg |
| Acesulfame K sweetener | 10 mg |
| E110 | 2 mg |
| Flavouring | 150 mg |

| 4) Sachets: | |
|---|---|
| VR-456 | 240 mg |
| Citicoline | 400 mg |
| Saccharose | 2265 mg |
| Citric acid | 50 mg |
| Silicon dioxide | 20 mg |
| Flavouring | 150 mg |
| Acesulfame K | 15 mg |

| 5)Two-layer controlled-release tablets | |
|---|---|
| NORMAL-RELEASE LAYER | |
| VR-456 | 120 mg |
| Leucocyanidin from *Vitis vinifera* | 100 mg |
| *Melilotus officinalis* (17-22%) | 50 mg |
| Dicafos | 304 mg |
| Aerosil | 3 mg |
| Vegetable magnesium stearate | 6 mg |
| Colouring | 1 mg |
| SLOW-RELEASE LAYER | |
| VR-456 | 240 mg |
| Leucocyanidins from *Vitis vinifera* | 150 mg |
| *Melilotus officinalis* (17-22%) | 100 mg |
| Metholose | 80 mg |
| Aerosil | 2 mg |
| Vegetable magnesium stearate | 3 mg |
| Microcel | 80 mg |
| Dicafos | 164 mg |

| 6) Oro-dispersible formulation | |
|---|---|
| VR-456 | 120 mg |
| Sorbitol | 160 mg |
| Orange flavouring | 20 mg |
| Mandarin flavouring | 5 mg |
| Acesulfame K | 2 mg |
| Aerosil | 5 mg |
| Fructose | 1566 mg |

| 7) gum disc | |
|---|---|
| VR-456 | 120 mg |
| Gum base | 800 mg |
| Aspartame | 2 mg |
| Menthol | 2 mg |
| Acesulfame | 1 mg |
| Levilite | 20 mg |
| Talc F.U. | 20 mg |
| Vegetable magnesium stearate | 18 mg |
| Shellac | 12 mg |
| Xylitol | 250 mg |
| Gum arabic | 6 mg |
| Titanium dioxide | 6 mg |
| Carnauba wax | 0,2 mg |

## Claims

1. *Ginkgo biloba* extract substantially devoid of PAF-antagonist activity.

2. Extract as claimed in claim 1, substantially devoid of the terpenic fraction.

3. Extract as claimed in claim 2, wherein the terpene content is below 0.1%.

4. Extract as claimed in claim 3, wherein the terpene content is below 0.01%.

5. Extract as claimed in claim 4, wherein the terpene content is below 0.001%.

6. Extract as claimed in claim 1, **characterised by** a ginkgo flavone glycoside content between 22 and 26%, under 5 ppm of ginkgolic acids and under 0.1% of total terpenes.

7. Extract as claimed in any of one claims 1 to 6, obtainable by repeated extractions of dried *Ginkgo biloba* leaves with a conventional system followed by repeated extractions with a solvent selected from alkyl esters, hypercritical CO₂, hexane, ethanol-water mixtures with an ethanol content of between 70 and 95% v/v, or butyl acetate, CH₂Cl₂ and methyl ethyl ketone.

8. Extract as claimed in claim 7, wherein the repeated extractions are performed with ethyl acetate.

9. Extract as claimed in any of one claims 1 to 8 with the addition of maltodextrins, dietary fibres, cellulose, sugars and/or polyols.

10. Pharmaceutical, nutraceutical or dietary compositions including the extract described in claims 1-9 as active ingredient.

11. Use of the extract claimed in claims 1-9 for the preparation of compositions for the vasculokinetic, antigeriatric, antidementia, procognitive or prosensory treatment in combination with antiaggregant and/or anticoagulant treatments.

12. Use as claimed in claim 11, in combination with other compounds having complementary or synergic activity.

13. Use as claimed in claim 11 or 12, for the geriatric treatment.

14. Use as claimed in claims 1 to 13, wherein the preparation takes the form of complexes such as liposome, phytosome, co-ground mixtures or terclatrate.
